(19) Europäisches Patentamt
European Patent Office
Office européen des brevets

(11) Publication number: **0 142 384 B1**

(12) **EUROPEAN PATENT SPECIFICATION**

(45) Date of publication of patent specification: **02.09.87**

(51) Int. Cl.[4]: **C 07 C 149/46,** C 08 G 59/68

(21) Application number: **84307982.3**

(22) Date of filing: **16.11.84**

(54) **A method for the preparation of photoinitiators.**

(30) Priority: **17.11.83 GB 8330692**

(43) Date of publication of application:
**22.05.85 Bulletin 85/21**

(45) Publication of the grant of the patent:
**02.09.87 Bulletin 87/36**

(84) Designated Contracting States:
**AT BE CH DE FR GB IT LI NL SE**

(56) References cited:
**GB-A-2 069 486**

(73) Proprietor: **Sericol Group Limited**
**24 Parsons Green Lane**
**London SW6 4HT (GB)**

(72) Inventor: **Ellwood, Michael**
**16, Chestnut Drive**
**Broadstairs Kent CT10 (GB)**

(74) Representative: **Claisse, John Anthony, Dr.**
**Burmah Oil Trading Ltd.**
**Castrol Research Laboratories**
**Whitchurch Hill**
**Pangbourne Reading Berkshire RG8 7QR (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 142 384 B1

Courier Press, Leamington Spa, England.

**Description**

Technical Field

Triarylsulphonium compounds are valuable as photoinitiators particularly for epoxy resin systems. Various methods have been described for their preparation but all have suffered from low yields and complex purification procedures.

Background Art

Thus in US patent 4197174 bis-[4-(diphenylsuphonio) phenyl] sulphide bishexafluorophosphate (formula 1 below) is prepared in very low yield (ca 5%) by chlorinating a solution of diphenylsulphide in dichloromethane in the presence of $AlCl_3$. The product is obtained by aqueous extraction followed by a complex procedure of sequential precipitation.

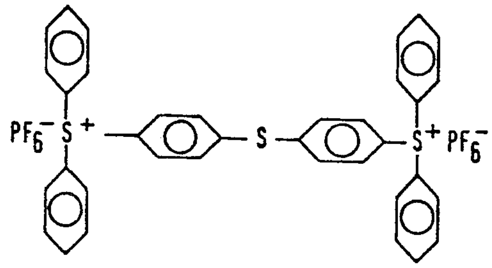

I

GB Patent 2069486 reveals a method of preparation whereby chlorine is introduced into a mixture of diphenylsulphide and $AlCl_3$ in the absence of any solvent.

The product thus prepared has the formula II:—

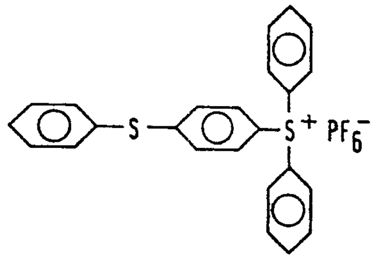

II

Yields of the final complex sulphonium salts obtained are of the order of 80%

A disadvantage of this method is the fact that the diarylsulphide is the only liquid reactant in the initial condensation reaction effectively also acting as a solvent. As the chlorine gas is bubbled through the reaction mix the viscosity progressively increases to such a point that the chlorine can no longer enter the reaction mix and a significant amount of the Lewis acid remains undissolved. This rapid viscosity increase effectively reduces the yield obtainable from this reaction step. A further disadvantage is the need for thorough purification of the resulting crude product to remove all traces of the strongly odoriferous diarylsulphide. If this reaction is carried out on a large scale the viscosity of the reaction mix is such that a very high torque stirrer is required for efficient agitation.

Summary of the Invention

Thus it is an object of this invention to provide a method of preparation of a compound of this type which does not suffer, or at least mitigates, the aforementioned problems. Thus the present invention provides a method for the preparation of a triaylsulphonium salt of the formula III:—

$$\begin{array}{c} \qquad\qquad\qquad Ar^1 \\ \qquad\qquad\qquad | \\ Ar^1{-}S{-}Ar^2{-}S^+\ X^- \\ \qquad\qquad\qquad | \\ \qquad\qquad\qquad Ar^1 \end{array}$$

III

wherein X may be $BF_4$, $PF_6$, $AsF_6$, $SbF_6$ or $SbF_5OH$; $Ar^1$ is a monovalent and $Ar^2$ a divalent aromatic radical each of which may be selected from phenyl, naphthyl, anthryl, thienyl, furanyl and pyrryl, these radicals optionally being substituted with one or more alkyl, substituted alkyl, alkoxy, cyano, nitro, acetamido or acyl groups or halogen atoms; which is characterised in that it comprises forming a triarylsulphonium halide in an initial reaction between a diarylsulphide $Ar^1{-}S{-}Ar^1$, a Lewis acid catalyst and a halogen in the presence of from 0.2 to 2 parts by weight of an inert solvent per part of diarylsulphide and at a temperature between 0°C and 25°C followed by an aqueous phase anion exchange between the triarylsulphonium halide and a compound of the formula NaX, KX or HX, wher X is as defined above, and then separation and purification of the resultant sulphonium salt.

Preferably the initial reaction mixture containing the triarylsulphonium halide is poured directly into substantially an equal weight of a mixture of ice and water. This has the effect of hydrolysing the Lewis acid complex present.

Said initial reaction is preferably accomplished at a temperature of between 10° and 15°C. This condensation reaction can be performed at ambient temperature or above but the yield obtained at such temperatures is lower than that obtained if the reaction is carried out in an ice bath within the range 0 to 25°C or as mentioned above preferably 10 to 15°C.

Typical inert solvents which may be used in the present invention include for example dichloromethane, carbon disulphide, nitrobenzene, or nitromethane. The preferred solvent is dichloromethane which is nonflammable, has relatively low toxicity and is easily removable due to its low boiling point. The amount of solvent utilised should be sufficient to provide solubility of the diarylsulphide, 0.2 to 2 parts by weight of solvent per part of diarylsulphide is desirable and preferably 0.25 to 1 parts per part. The solvent is inert only to the extent that it does not react significantly with the diarylsulphide or the Lewis acid, the latter, of course, being extremely reactive and thus limiting the selection of "inert" solvents.

The Lewis acid catalyst may be selected from those well known for their efficiency in Friedel Craft reactions, such as aluminium chloride or bromide, boron trifluoride or trichloride, ferric chloride, stannic chloride, phosphorus pentafluoride, arsenic pentafluoride or antimony pentafluoride. The catalyst is added with agitation. The amount of catalyst employed may be 0.1 to 5 moles per mole of diarylsulphide and preferably 0.25 to 1 moles per mole.

It is preferred that elemental halogen is added in the initial reaction, preferably at such a rate that the reaction temperature does not exceed 15°C whilst maintaining agitation. It is preferable to add from 0.2 to 2 moles of elemental halogen per mole of diarylsulphide.

After addition of the halogen the reaction mix may be stirred at 10—15°C for a further 0.5 to 2 hours. Although the sulphonium chloride, bromide or iodide thus prepared in said initial reaction may be isolated by conventional techniques, it is nevertheless preferable to continue the preparation of the end product with the intermediate product retained in an aqueous phase.

To the aqueous reaction mix maintained at a temperature of from 20° to 100°C is added, with agitation, the sodium or potassium salt of fluoroboric acid, hexafluorophosphoric acid, hexafluoroarsenic acid or hexafluoroantimonic acid. The free acids themselves may be employed in this invention but this is not preferred due to their extremely corrosive natures. These salts are added in essentially equimolar proportions with respect to the sulphonium chloride, bromide or iodide, any excess merely increasing the costs of the process.

The polyhalogenometal or metalloid sulphonium salt is then recovered by decantation or filtration. The essentially pure product is washed with water and preferably an organic solvent in which the product is insoluble, such as diethylether, isopropyl alcohol or petroleum spirit. The purified product is then dried under vacuum at 40—100°C.

In order to illustrate the various aspects of the present invention the following non-limiting examples are detailed. All parts are by weight unless otherwise specified.

### Example 1

500 parts diphenylsulphide were admixed with 260 parts dichloromethane in a glass reaction vessel cooled in an ice bath. 200 parts ground aluminium chloride were added with stirring, chlorine gas was then rapidly bubbled through the reaction mix for 3 hours during which time the temperature was maintained at 13—15°C. After this the weight of the reaction flask and contents had increased by 50 parts. The contents of the flask were then poured onto 1,000 parts of ice.

The resultant creamy paste was heated to 40°C and 270 parts of potassium hexafluorophosphate were added portion wise with agitation, the whole was then heated on a steam bath to remove the dichloromethane. The solid product was isolated by decantation, washed twice with water and twice with diethyl ether, then dried under vacuum at 40°C. The product was identified as 4-thiophenoxytriphenylsulphonium hexafluorophosphate by virtue of its IR, UV and $C^{13}$NMR spectra. There was obtained 683 parts of this product which represents 98% of theoretical yield based on the diphenylsulphide. The above procedure was repeated in the absence of any solvent. The product thus obtained was a white sticky solid, 435 parts representing 63% of theoretical yield based on diphenylsulphide. Confirmation of the product as 4-thiophenoxytriphenylsulphonium hexafluorophosphate was achieved by IR and UV spectroscopy. This example illustrates the much higher yields obtainable using a small amount of an inert solvent.

### Example 2

35 parts of ground aluminium chloride were added to a solution of 100 parts diphenylsulphide in 66 parts dichloromethane. The reaction was maintained at 15°C whilst 43 parts of bromine were added portionwise. After stirring for a further 4 hours the mixture was poured onto 500 parts of ice, the whole was then heated to 50°C and 60 parts of potassium hexafluorophosphate were added. The resultant cream coloured semi-solid was washed twice with warm water and isopropyl alcohol and dried under vacuum at 40°C. 133 parts of a white solid were obtained being 97% of theoretical yield based on diphenylsulphide; the product was identified as 4-thiophenoxytriphenylsulphonium hexafluorophosphate from its IR, UV and $C^{13}$ NMR spectra.

Example 3

An example illustrating the use of the above prepared sulphonium salt as a cationic photoinitiator for epoxy resins is given below.

A 3% solution of 4-thiophenoxytriphenylsulphonium hexafluorophosphate in 3,4-expoxy — cyclohexylmethyl — 3,4-cyclohexanecarboxylate (sold under the designation CY 179 by Ciba Geigy) was draw coated onto a steel panel. This was passed through a water cooled, Colordry UV dryer (400 W/in) at 135 ft/min resulting in a tack free coating.

Example 4

100 parts of diphenylsulphide were admixed with 20 parts of carbon disulphide in a glass reaction vessel cooled in an ice bath. 40 parts of ground aluminium chloride were added with stirring, chlorine gas was then bubbled through the reaction mix for 7 hours at such a rate that the temperature was maintained at 13—18°C. After this time the weight of the reaction vessel and its content had increased by 15 parts and no undissolved aluminium chloride was present. The contents of the reaction vessel were then poured onto 500 part of ice with stirring, a further 1000 parts of water were then added and the mixture was heated to 40°C. 60 parts potassium hexafluorophosphate were added portionwise with stirring and a white precipitate was formed. The aqueous layer was decanted off and the resultant semi solid washed twice with 1000 parts of warm water and filtered. The product was washed once with cold isopropanol and twice with ether and dried under vacuum at 60°C. 118 parts of a cream coloured solid were obtained 87% theoretical yield based on diphenylsulphide, the product was identified as 4-thiophenoxytriphenylsulphonium hexafluorophosphate from its IR and UV spectra.

Method A

50 parts ground aluminium chloride were added to a solution of 100 parts diphenylsulphide in 400 parts dichloromethane. The reaction mix was maintained at 13—16°C for 5 hours during which time chlorine was sparged into the system. After this addition the weight of the reaction flask and contents had increased by 30 parts. The reaction mixture was poured onto 500 parts of ice with stirring and a further 1000 parts of water were added. The whole was heated to 40°C and 60 parts potassium hexafluorophosphate were added portionwise with stirring. The resultant semi solid was washed twice with warm water, once with cold isopropanol and twice with ether, filtered, and dried at 60°C under vacuum. 109 parts of a white solid were obtained, this was identified as 4-thiophenoxytriphenylsulphonium hexafluorophosphate by virtue of its IR and UV spectra.

Method A illustrates the use of a large excess of solvent and demonstrates that none of the product which would be expected from USP 4197174 (Formula I) was found.

**Claims**

1. A method for the preparation of a triarylsulphonium salt of the formula III:—

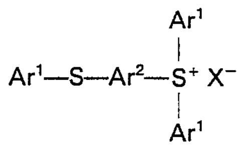

III

wherein X may be $BF_4$, $PF_6$, $AsF_6$, $SbF_6$ or $SbF_5OH$; $Ar^1$ is a monovalent and $Ar^2$ a divalent aromatic radical each of which may be selected from phenyl, naphthyl, anthryl, thienyl, furanyl and pyrryl, these radicals optionally being substituted with one or more alkyl, substituted alkyl, alkoxy, cyano, nitro, acetamido or acyl groups or halogen atoms; which method is characterised in that it comprises forming a triarylsulphonium halide in an initial reaction between a diarylsulphide $Ar^1$—S—$Ar^1$, a Lewis acid catalyst and a halogen in the presence of from 0.2 to 2 parts by weight of an inert solvent per part of diarylsulphide and at a temperature between 0°C and 25°C followed by an aqueous phase anion exchange between the triarylsulphonium halide and a compound of the formula NaX, KX or HX, where X is as defined above, and then separation and purification of the resultant sulphonium salt.

2. A method as claimed in claim 1 wherein the initial reaction mixture containing the triarylsulphonium halide is poured directly into substantially an equal weight of a mixture of ice and water.

3. A method as claimed in claim 1 wherein the initial reaction is carried out at a temperature between 10°C and 15°C.

4. A method as claimed in any of claims 1 to 3 wherein the inert solvent is dichloromethane, carbon disulphide, nitrobenzene or nitromethane.

5. A method as claimed in any of claims 1 to 4 wherein the amount of solvent is 0.25 to 1 parts by weight per part of diarylsulphide.

6. A method as claimed in any of claims 1 to 5 wherein the inert solvent is dichloromethane.

7. A method as claimed in any of claims 1 to 6 wherein the Lewis acid is anhydrous aluminium chloride.

8. A method as claimed in any of claims 1 to 7 wherein the diarylsulphide is diphenylsulphide.

9. A method as claimed in any of claims 1 to 8 wherein the halogen is chlorine, bromine or iodine.

**Patentansprüche**

1. Verfahren zur Herstellung eines Triarylsulfoniumsalzes der Formel III:

$$\begin{array}{r@{}l} & \quad\; Ar^1 \\ & \quad\;\; | \\ Ar^1—S—Ar^2—& S^+ X^- \\ & \quad\;\; | \\ & \quad\; Ar^1 \end{array} \qquad \text{III}$$

worin X $BF_4$, $PF_6$, $AsF_6$, $SbF_6$, oder $SbF_5OH$ sein kann, $Ar^1$ einen monovalenten und $Ar^2$ einen divalenten aromatischen Rest aus der Gruppe der Phenyl-, Naphthyl-, Anthryl-, Thienyl-, Furanyl- und Pyrryl-reste bedeuten, wobei diese Reste gewünschtenfalls durch eine oder mehrere Alkyl-, substituierte Alkyl-, Alkoxy-, Cyan-, Nitro-, Acetamido- oder Acylgruppen oder Halogenatome substituiert sein können, dadurch gekennzeichnet, dass in einer Initialreaktion zwischen einem Diarylsulfid $Ar^1—S—Ar^1$, einem Lewis-säure-Katalysator und einem Halogen, in Gegenwart von 0,2 bis 2 Gewichtsteilen eines inerten Lösungsmittels pro Teil Diarylsulfid und bei einer Temperatur zwischen 0°C und 25°C, ein Triarylsulfonium-halogenid gebildet, wird, danach in wässriger Phase ein Anionenaustausch zwischen dem Triarylsulfonium-halogenid und einer Verbindung der Formel NaX, KX oder HX, worin X die oben angegebene Bedeutung hat, durchgeführt und dann das so erhaltene Sulfoniumsalz abgetrennt und gereinigt wird.

2. Verfahren nach Anspruch 1, dadurch gekennzeichnet, dass die initiale Reaktionsmischung, die das Triarylsulfonium-halogenid enthält, unmittelbar in praktisch die gleiche Gewichtsmenge einer Mischung von Eis und Wasser gegossen wird.

3. Verfahren nach Anspruch 1, dadurch gekennzeichnet, dass die Initialreaktion bei einer Temperatur zwischen 10°C und 15°C durchgeführt wird.

4. Verfahren nach einem der Ansprüche 1 bis 3, dadurch gekennzeichet, dass als inertes Lösungsmittel Dichlormethan, Schwefelkohlenstoff, Nitrobenzol oder Nitromethan verwendet wird.

5. Verfahren nach einem der Ansprüche 1 bis 4, dadurch gekennzeichnet, dass das Lösungsmittel in einer Menge von 0,25 bis 1 Gewichtsteil pro Teil Diarylsulfid verwendet wird.

6. Verfahren nach einem der Ansprüche 1 bis 5, dadurch gekennzeichnet, dass als inertes Lösungsmittel Dichlormethan verwendet wird.

7. Verfahren nach einem der Ansprüche 1 bis 6, dadurch gekennzeichnet, dass als Lewis-säure wasserfreies Aluminiumchlorid verwendet wird.

8. Verfahren nach einem der Ansprüche 1 bis 7, dadurch gekennzeichnet, dass als Diarylsulfid Diphenylsulfid verwendet wird.

9. Verfahren nach einem der Ansprüche 1 bis 8, dadurch gekennzeichnet, dass als Halogen Chlor, Brom oder Jod verwendet wird.

**Revendications**

1. Procédé pour la préparation d'un sel de triaryl-sulfonium de formule III:

$$\begin{array}{r@{}l} & \quad\; Ar^1 \\ & \quad\;\; | \\ Ar^1—S—Ar^2—& S^+ X^- \\ & \quad\;\; | \\ & \quad\; Ar^1 \end{array} \qquad \text{III}$$

dans laquelle X peut être $BF_4$, $PF_6$, $AsF_6$, $SbF_6$ ou $SbF_5OH$; $Ar^1$ est un radical aromatique monovalent et $Ar^2$ est un radical aromatique divalent dont chacun peut être choisi parmi phényle, naphtyle, anthryle, thiényle, furyle et pyrryle, ces radicaux étant facultativement substitués par un ou plusieurs groupes alkyles, alkyles substitués, alcoxy, cyano, nitro, acétamido ou acyles ou atomes d'halogène; lequel procédé est caractérisé en ce qu'il comprend la formation d'un halogènure de triarylsulfonium dans une réaction initiale entre un sulfure de diaryle $Ar^1—S—Ar^1$, un acide de Lewis catalytique et un halogène en présence de 0,2 à 2 parties en poids d'un solvant inerte par partie de sulfure de diaryle et à une température entre 0°C et 25°C, suivie d'un échange d'anions en phase aqueuse entre l'halogénure de triarylsulfonium et un composé de formule NaX, KX ou HX dans laquelle X est comme défini ci-dessus, puis la séparation et la purification du sel de sulfonium obtenu.

2. Procédé selon la revendication 1 dans lequel le mélange réactionnel initial contenant l'halogénure de triarylsulfonium est versé directement dans un poids pratiquement égal d'un mélange de glace et d'eau.

3. Procédé selon la revendication 1 dans lequel la réaction initiale est effectuée à une température entre 10°C et 15°C.

4. Procédé selon l'une quelconque des revendications 1 à 3 dans lequel le solvant inerte est le dichlorométhane, le disulfure de carbone, le nitrobenzène ou le nitrométhane.

5. Procédé selon l'une quelconque des revendications 1 à 4 dans lequel la quantité de solvant est de 0,25 à 1 partie en poids par partie de sulfure de diaryle.

6. Procédé selon l'une quelconque des revendications 1 à 5 dans lequel le solvant inerte est le dichlorométhane.

7. Procédé selon l'une quelconque des revendications 1 à 6 dans lequel l'acide de Lewis est le chlorure d'aluminium anhydre.

8. Procédé selon l'une quelconque des revendications 1 à 7 dans lequel le sulfure de diaryle est le sulfure de diphényle.

9. Procédé selon l'une quelconque des revendications 1 à 8 dans lequel l'halogène est le chlore, le brome ou l'iode.